(19) Europäisches Patentamt
European Patent Office
Office européen des brevets

(11) Veröffentlichungsnummer: **0 234 380 A2**

(12) **EUROPÄISCHE PATENTANMELDUNG**

(21) Anmeldenummer: **87101744.8**

(22) Anmeldetag: **09.02.87**

(51) Int. Cl.4: **C07D 213/81**, C07B 43/06, C07D 215/48, C07D 217/26

(30) Priorität: **20.02.86 DE 3605423**

(43) Veröffentlichungstag der Anmeldung: **02.09.87 Patentblatt 87/36**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Marzolph, Gerhard, Dr.**
**Semmelweiss-Strasse 87a**
**D-5000 Köln 80(DE)**
Erfinder: **Streicher, Willi, Dr.**
**Andreas-Gryphius-Strasse 7**
**D-5000 Köln 80(DE)**
Erfinder: **Blank, Heinz Ulrich, Dr.**
**Am Geusfelde 35**
**D-5068 Odenthal(DE)**

(54) **Verfahren zur Herstellung von Pyridin-carbonsäure-N-tert.-alkylamiden.**

(57) Pyridin-carbonsäure-N-tert.-alkylamide können durch Umsetzung eines Pyridincarbonsäurenitrils mit einem tertiäre Carbeniumionen liefernden Alkylierungsmittel in Gegenwart von 50-100 %iger Schwefelsäure bei 0-100° C und in weiterer Gegenwart von aliphatischen oder aromatischen Kohlenwasserstoffen oder aliphatischen oder aromatischen Halogenkohlenwasserstoffen hergestellt werden.

EP 0 234 380 A2

## Verfahren zur Herstellung von Pyridin-carbonsäure-N-tert.-alkylamiden

Die vorliegende Erfindung betrifft ein Verfahren zur Herstellung von Pyridin-carbonsäure-N-tert.-alkylamiden durch Umsetzung von Pyridincarbonsäurenitrilen (Cyanopyridinen) mit tertiäre Carbeniumionen liefernden Alkylierungsmitteln in Gegenwart von Schwefelsäure und in weiterer Gegenwart von aliphatischen oder aromatischen Kohlenwasserstoffen oder aliphatischen oder aromatischen Halogenkohlenwasserstoffen.

N-tert.-Alkylamide von aliphatischen und aromatischen Carbonsäuren sind durch Umsetzung der entsprechenden aliphatischen oder aromatischen Nitrile beispielsweise mit tertiären Alkoholen unter dem Einfluß starker Säuren wie Schwefelsäure in der als "Ritter-Reaktion" bekannten Umsetzung zugänglich (Organic Reactions 17, 213 ff. (1969)).

Neben der die Umsetzung bewirkenden Schwefelsäure ist vielfach auch zusätzlich zur Schwefelsäure Essigsäure als Lösungsmittel verwendet worden. Es ist auch versucht worden, andere Lösungsmittel als Essigsäure einzusetzen. Hierzu zählen zunächst die Versuche, den Überschuß eines Reaktionspartners als Lösungsmittel zur verwenden. Beispielsweise ist in Bull. Soc. Chim. France 1972, Seite 189 die Umsetzung von Cyanhydrinen des Butyraldehyds und des Dibenzylacetaldehyds mit überschüssigem tert.-Butanol beschrieben. Ferner findet sich in J. Chem. Soc. (London) 1967, Seite 1558, besonders Seite 1562 die Umsetzung von 1-Acetyl-4-amino-3-cyano-1,2,5,6-tertrahydropyridin mit überschüssigem tert.-Butylacetat unter dem Einfluß von 70 %iger Perchlorsäure in einer mehr als 16-stündigen Reaktion bei 10°C; die gleiche Literaturstelle nennt auf Seite 1563 die Reaktion von 3-Cyanopyridin mit mehr als 10-facher molarer Menge an tert.-Butylacetat ebenfalls unter Zusatz von Perchlorsäure in einer 18-stündigen Reaktion bei 20°C. Die langen Reaktionszeiten, die sehr großen Überschüsse der teuren Reaktionskomponente tert.-Butylacetet, die zu tert.-Butanol und Essigsäure verseift wird, und der Einsatz der technisch unerwünschten Perchlorsäure sind Nachteile dieses Verfahrens.

Im Falle des Acetonitrils als der Nitrilkomponente in der Ritter-Reaktion wurde auch ein Überschuß hiervon bereits als Lösungsmittel untersucht (Bull. Soc. Chim. France, 1966, Seite 1299, besonders Seite 1300).

Daneben berichtet Organic Reaktions (Loc. cit.) auch über Versuche, andere Lösungsmittel einzusetzen. Von diesen Lösungsmitteln werden jedoch die nicht solvatisierend und polarisierend wirkenden als nicht brauchbar angesehen. Geht man den angegebenen Quellen nach (Bull. Soc. Chim. France, 1966, Seite 1299 ff., bereits erwähnt, und Bull. Soc. Chim. France 1966, Seite 1376 ff.), so erfährt man, daß die Ausbeuten bei Verwendung anderer Lösungsmittel grundsätzlich schlechter sind als die Ergebnisse, die man bei Verwendung von Essigsäure oder ganz ohne Lösungsmittel (d.h. nur in Gegenwart von $H_2SO_4$) erhält. Von 98,4 % Ausbeute (Essigsäure/$H_2SO_4$) bzw. 90 % Ausbeute ($H_2SO_4$ ohne weiteres Lösungsmittel) gehen die Ausbeuten bei der Verwendung von Dibutylether bzw. para-Dioxan auf Werte zwischen 65 und 86 % zurück; bei Verwendung unpolarer Lösungsmittel, wie Tetrachlorkohlenstoff, n-Heptan oder n-Hexan, gehen die Ausbeuten drastisch auf Werte von 10 bis 15 % zurück.

Die Umsetzung von Pyridincarbonsäurenitrilen (Cyanopyridinen) in der Ritter-Reaktion ist nun durch die Salzbildung am Pyridin-N-Atom komplizierter als bei anderen Substraten. Die hierbei auftretende Neutralisationswärme erschwert die Beherrschung des Reaktionsablaufs. Überhitzte Stellen im Reaktionsansatz können sodann zur Verseifung der Nitrilgruppe führen; weiterhin ist die Abspaltung des tert.-Alkylrestes vom N-Atom von bereits gebildeten Pyridin-carbonsäure-N-tert.-alkylamiden in Form des zugehörigen Olefins zu befürchten. Die Ritter-Reaktion an Cyanopyridinen findet sich daher in der Literatur nur sehr spärlich und ist nur unter Anlegung spezieller Reaktionsbedingungen untersucht.

Hierzu zählt die bereits genannte Literaturstelle J. Chem. Soc. (London) 1967, Seite 1558 und weiterhin J. Am. Chem. Soc. 1952, Seite 763, wo 3-Cyanopyridin mit $\alpha,\alpha$-Dimethyl-$\beta$-phenethyl-alkohol in Gegenwart von konzentrierter Schwefelsäure und Zusatz von Eisessig als Lösungsmittel umgesetzt wird. Die Ausbeute in der zuletzt genannten Reaktion beträgt 71 %. Die genannte Arbeitsweise hat weiterhin den Nachteil, daß das Nitril nur in 18 %iger Lösung umgesetzt wird, wodurch die Raum-Zeit-Ausbeute negativ beeinflußt wird.

Es wurde nun überraschend gefunden, daß bei der Umsetzung von Cyanopyridinen mit tertiäre Carbeniumionen liefernden Alkylierungsmitteln unter dem Einfluß von Schwefelsäure die Gegenwart von aliphatischen oder aromatischen Kohlenwasserstoffen oder aliphatischen oder aromatischen Halogenkohlenwasserstoffen keine Minderung der Ausbeute bewirkt, sondern daß in vorteilhafter Weise alle Probleme, die mit der Salzbildung am Pyridin-N-Atom verbunden sind, beherrscht werden können, so insbesondere das Problem der Rührfähigkeit eines Reaktionsansatzes und das Problem einer guten Temperaturkontrolle.

Es wurde nun ein Verfahren zur Herstellung von Pyridin-N-tert.-alkylamiden der Formel

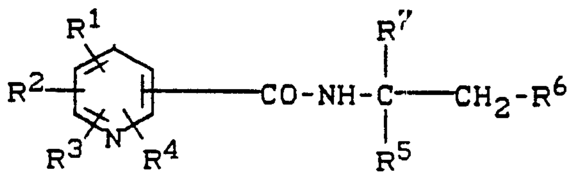 (I),

in der
$R^1$, $R^2$, $R^3$ und $R^4$ unabhängig voneinander Wasserstoff, Halogen, Alkyl, Alkoxy, Phenyl, Benzyl oder Nitrol bedeuten und zwei der Reste $R^1$ bis $R^4$ gemeinsam einen anellierten Benzolring bedeuten können und
$R^5$, $R^6$ und $R^7$ unabhängig voneinander geradkettige oder verzweigte Alkylgruppen mit 1-8 C-Atomen bedeuten, wobei weiterhin $R^5$ und $R^6$ gemeinsam mit den C-Atomen, die sie substituieren, einen aliphatischen Ring von 5-8 Ringgliedern bilden können, $R^7$ weiterhin ein gegebenenfalls substituierter Phenylrest sein kann und $R^6$ außerdem Wasserstoff sein kann,
durch Umsetzung eines Pyridincarbonsäurenitrils der Formel

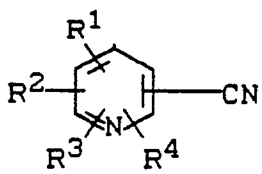 (II),

in der
$R^1$ bis $R^4$ die angegebene Bedeutung besitzen,
mit einem tertiäre Carbeniumionen liefernden Alkylierungsmittel der Formel

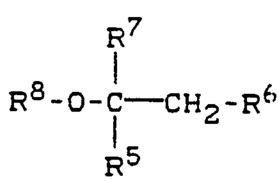 (III),

oder der Formel

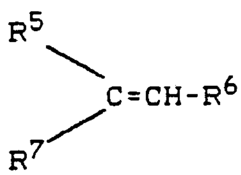 (IV),

in denen
$R^5$ bis $R^7$ die angegebene Bedeutung besitzen und
$R^8$ Wasserstoff, Alkyl oder Acyl bedeutet,
in Gegenwart von Schwefelsäure gefunden, das dadurch gekennzeichnet ist, daß die Reaktion bei 0-100°C in Gegenwart eines aliphatischen oder aromatischen (Halogen)Kohlenwasserstoffes durchgeführt wird.

Als Halogen sei beispielsweise Fluor, Chlor, Brom oder Iod, bevrozugt Fluor, Chlor oder Brom, genannt.

Als Alkyl seien beispielsweise geradkettige oder verzweigte gesättigte Kohlenwasserstoffreste mit 1-8, bevorzugt 1-4, besonders bevorzugt 1-2 C-Atomen genannt, wie Methyl, Ethyl, Propyl, Isopropyl, Butyl, Isobutyl, Hexyl, Octyl. Alkyl kann 1-oder mehrfach mit Fluor oder Chlor substituiert sein, beispielsweise als Fluormethyl, Trifluormethyl, Chlormethyl, Dichlormethyl, Trichlormethyl, Perfluorethyl, Perchlorethyl und ihre Homologen.

Als Alkoxy seinen Reste von Alkanolen mit 1-8, bevorzugt 1-4, besonders bevorzugt 1-2 C-Atomen genannt; die im Alkoxy vorkommenden Alkylgruppen sind die gleichen, die oben genannt wurden.

Wenn zwei der Reste $R^1$ bis $R^4$ einen anellierten Benzolring bedeuten, gelangt man in die Reihe der Chinolin-oder Isochinolin-Verbindungen.

Die Reste $R^5$ und $R^6$ können mit den durch sie substituierten C-Atomen einen aliphatischen Ring mit 5-8, bevorzugt 5-6 Ringgliedern bilden.

Als Acyl sei solches mit 2-7, bevorzugt 2-4 C-Atomen, genannt; besonders bevorzugtes Acyl ist Acetyl.

Als Cyanopyridine seien bevorzugt solche der Formel

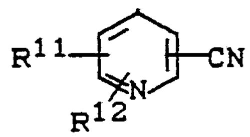

(V)

genannt, in der

$R^{11}$ und $R^{12}$ unabhängig voneinander Wasserstoff, Halogen, Alkyl, Alkoxy oder Nitro bedeuten.

Als Cyanopyridine seien besonders bevorzugt solche der Formel

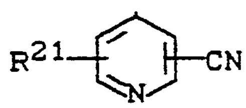

(VI)

genannt, in der

$R^{21}$ Wasserstoff, Halogen, Alkyl, Alkoxy oder Nitro bedeutet.

In ganz besonders bevorzugter Weise bedeutet $R^{21}$ Wasserstoff, Fluor, Chlor, Brom, Methyl, Ethyl, Methoxy, Ethoxy oder Nitro.

Als Alkylierungsmittel der Formeln (III) und (IV) können tertiäre Alkohole, Ether oder Ester solcher tertiären Alkanole oder an der Doppelbindung verzweigte iso-Alkene genannt werden. Allen genannten Verbindungsklassen gemeinsam ist ihre Fähigkeit zur Bildung von Carbeniumkationen der Formel

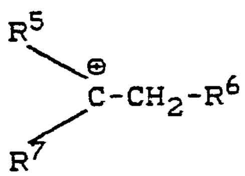

(VII)

mit den angegebenen Bedeutungen für $R^5$ bis $R^7$.

Als bevorzugte Alkylierungsmittel seien solche der Formeln

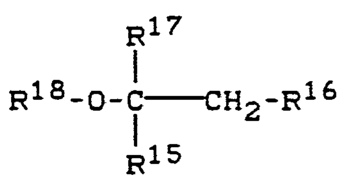

oder

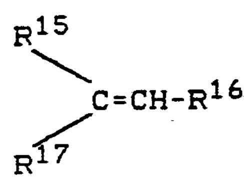

(VIII) (IX)

genannt, in denen

$R^{15}$ und $R^{17}$ unabhängig voneinander geradkettiges $C_1$-$C_4$-Alkyl bedeuten,

$R^{16}$ für Wasserstoff oder geradkettiges oder verzweigtes $C_1$-$C_4$-Alkyl steht und

$R^{18}$ Wasserstoff, $C_1$-$C_4$-Alkyl oder Acetyl bedeutet,

wobei weiterhin $R^{15}$ und $R^{18}$ gemeinsam mit den C-Atomen, die sie substituieren, einen aliphatischen Ring von 5-6 Ringgliedern bilden können.

In ganz besonders bevorzugter Weise bedeuten $R^{15}$, $R^{16}$ und $R^{17}$ unabhängig voneinander Methyl oder Ethyl, $R^{16}$ kann zusätzlich Wasserstoff bedeuten und $R^{18}$ bedeutet Wasserstoff, Methylk, Ethyl oder Acetyl.

Als Beispiele für Alkylierungsmittel seien genannt: tert.-Butanol, tert.-Amylalkohol, Methyl-tert.-butylether, Methyl-tert.-amylether, tert.-Butylacetat, tert.-Amylacetat, Isobuten, tert.-Amylen, 1-Methylcyclopent-1-en, 1-Methylcyclohex-1-en, 1-Methylcyclopentanol-1-oder 1-Methyl-cyclohexanol-1.

Das Mengenverhältnis des Alkylierungsmittels zum Cyanopyridin kann weitgehend beliebig gewählt werden, beispielsweise 0,8-4 Mol, bevorzugt 0,8-2,5 Mol, besonders bevorzugt 1,0-2,0 Mol Alkylierungsmittel je Mol des Cyanopyridins.

Zur Durchführung des erfindungsgemäßen Verfahrens wird 50-100 %ige, bevorzugt 60-96 %ige Schwefelsäure in einer Menge von beispielsweise 0,5-3 Mol, bevorzugt 0,8-2,7 Mol, besonders bevorzugt 1,0-2,5 Mol pro Mol des Cyanopyridins eingesetzt.

Das erfindungsgemäße Verfahren wird bei einer Temperatur von 0-100° C, bevorzugt 10-95° C, besonders bevorzugt 20-90° C, durchgeführt. Im Rahmen des genannten Temperaturbereichs kann die Reaktionstemperatur während der Reaktion erhöht oder erniedrigt werden.

Das erfindungsgemäße Verfahren wird in Gegenwart von 10-2000 ml, bevorzugt 20-1000 ml, besonders bevorzugt 30-300 ml und ganz besonders bevorzugt 40-200 ml eines aliphatischen oder aromatischen Kohlenwasserstoffs oder aliphatischen oder aromatischen Halogenkohlenwasserstoffs pro Mol des Cyanopyridins durchgeführt.

Als aliphatische Kohlenwasserstoffe seien beispielsweise geradkettige, verzweigte oder cyclische Kohlenwasserstoffe mit 5-12, bevorzugt 6-8 C-Atomen genannt, beispielsweise die isomeren Pentane, Hexane, Heptane, Octane, Decane, Dodecane, Cyclopentan, Methyl-cyclopentan, Cyclohexan, Methyl-cyclohexan, Cycloheptan oder Cyclooctan.

Als aromatische Kohlenwasserstoffe seien beispielsweise Benzol, Toluol oder die isomeren Xylole genannt.

Als aliphatische Halogenkohlenwasserstoffe seien beispielsweise geradkettige, verzweigte oder cyclische Kohlenwasserstoffe mit 1-8, bevorzugt 1-4 C-Atomen genannt, die 1-oder mehrfach mit Fluor, Chlor oder Brom, bevorzugt Fluor oder Chlor, besonders bevorzugt mit Chlor substituiert sind, beispielsweise Methylenchlorid, Chloroform, Tetrachlorkohlenstoff, Ethylendichlorid, Tetrachlorethan, 1-oder mehrfach chlorierte Propane, Butane, Hexane oder Octane sowie die entsprechenden Fluor-oder Brom-Verbindungen. Selbstverständlich sind auch gemischt halogenierte Aliphaten einsetzbar, ebenso wie fluorierte, chlorierte oder bromierte Cycloaliphaten.

Als aromatische Halogenkohlenwasserstoffe, seien beispielsweise Chlorbenzol, Brombenzol oder die isomeren Dichlorbenzole genannt.

In bevorzugter Weise seien industriell preiswert erhältliche (Halogen)Kohlenwasserstoffe genannt, wie Isododecan, Isooctan, Isopentan, Hexan, Heptan, Petrolether, 2,3-Dimethylbutan, 2,4-Dimethylpentan, Cyclohexan, Toluol, Xylol, Chlor-cyclohexan, Ethylendichlorid, 1,1,2,2-Tetrachlorethan, 1,1,1-Trichlorethan, 1,2-Dichlorpropan, Chloroform, Tetrachlorokohlenstoff, ortho-Öl, Methylen chlorid, Ethylidendichlorid und Chlorbenzol. Selbstverständlich können auch Gemische der genannten (Halogen)Kohlenwasserstoffe eingesetzt werden.

Das erfindungsgemäße Verfahren kann grundsätzlich diskontinuierlich, beispielsweise in einem Autoklaven, Druckkessel oder Rührkessel, oder auch kontinuierlich, beispielsweise in einem beheizbaren oder kühlbaren und drückfesten Strömungsrohr oder einer Kesselkaskade durchgeführt werden. Ein höherer Druck als der Normaldruck brauch nur angelegt zu werden, wenn niedrigsiedende Reaktionspartner in der flüssigen Phase gehalten werden sollen.

Für die Durchführung des erfindungsgemäßen Verfahrens sind verschiedene Varianten möglich. Grundsätzlich ist für die Zugabe des Cyanopyridins, des Alkylierungsmittels, der Schwefelsäure und des - (Halogen)Kohlenwasserstoffs eine beliebige Reihenfolge möglich. In bevorzugter Weise wird jedoch der (Halogen)Kohlenwasserstoff mindestens teilweise vorgelegt oder mit einem der Reaktionspartner gleichzeitig zugegeben, wobei die gleichzeitige Zugabe in Form einer Mischung oder als simultane Zugabe des - (Halogen)Kohlenwasserstoffs und des gewählten Reaktionspartners verstanden wird. Folgende Beispiele seien als Durchführungsvarianten genannt:

a) zu (mindestens teilweise) vorgelegtem (Halogen)Kohlenwasserstoff werden simultan das Cyanopyridin, das Alkylierungsmittel und die Schwefelsäure sowie gegebenenfalls restlicher (Halogen)-Kohlenwasserstoff zugegeben;

b) das Cyanopyridin, das Alkylierungsmittel und der (Halogen)Kohlenwasserstoff werden vorgelegt und $H_2SO_4$ wird zudosiert;

c) der (Halogen)Kohlenwasserstoff und $H_2SO_4$ werden vorgelegt, und das Cyanopyridin und das Alkylierungsmittel werden nacheinander oder simultan zudosiert;

d) $H_2SO_4$ und das Alkylierungsmittel sowie gegebenenfalls mindestens ein Teil des (Halogen)-Kohlenwasserstoffs werden vorgelegt, und das Cyanopyridin und der (gegebenenfalls restliche) (Halogen)-Kohlenwasserstoff werden zudosiert;

e) das Cyanopyridin und gegebenenfalls der (Halogen)Kohlenwasserstoff oder wenigstens ein Teil desselben werden vorgelegt, und $H_2SO_4$, das Alkylierungsmittel und, soweit nicht bereits vorgelegt, der -(Halogen)Kohlenwasserstoff werden zudosiert;

f) der (Halogen)Kohlenwasserstoff wird vorgelegt, $H_2SO_4$ und das Cyanopyridin werden nacheinander oder simultan zugefügt, und das Alkylierungsmittel wird zudosiert, wobei ein Teil des (Halogen)-Kohlenwasserstoffs gegebenenfalls im Gemisch mit dem Alkylierungsmittel zudosiert wird.

In bevorzugter Weise wird der gesamte (Halogen)Kohlenwasserstoff vorgelegt. In weiterer bevorzugter Weise wird zusätzlich zum (Halogen)Kohlenwasserstoff auch das Cyanopyridin vorgelegt, wobei sodann entweder zusätzlich das Alkylierungsmittel vorgelegt wird und $H_2SO_4$ zudosiert wird oder zusätzlich $H_2SO_4$ zum Gemisch aus (Halogen)Kohlen wasserstoff und Cyanopyridin zugegeben wird und anschließend das Alkylierungsmittel zudosiert wird.

Nach Beendigung der Reaktion wird die Phase des (Halogen)Kohlenwasserstoffs von der $H_2SO_4$-haltigen Phase abgetrennt. Die $H_2SO_4$-haltige Phase wird sodann neutralisiert. Zur Neutralisation können beispielsweise die Hydroxide oder Carbonate der Alkali-oder Erdalkalimetalle eingesetzt werden. Nach der Neutralisation kann im Falle von flüssigen Reaktionsprodukten die organische Phase des Reaktionsproduktes von der wäßrigen Phase abgetrennt werden; bei festen Reaktionsprodukten werden diese abgesaugt oder abgepreßt oder durch geeignete Extraktionsmittel aus dem Reaktionsgemisch isoliert und nach dem Entfernen des Extraktionsmittels beispielsweise durch Destillation gereinigt. Das so gewonnene Produkt fällt in hoher Reinheit und in sehr guten Ausbeuten an. Es ist jedoch auch möglich vor der Abtrennung der (Halogen)Kohlenwasserstoff-Phase die Neutralisation vorzunehmen. Im Falle geeigneter (Halogen)-Kohlenwasserstoffe geht sodann das Reaktionsprodukt in diese organische Phase über und kann nach einer Wäsche dieser Phase, beispielsweise mit Hydrogencarbonatlösung in bekannter Weise durch Destillation oder Kristallisation gewonnen werden.

Pyridin-carbonsäure-N-tert.-alkylamide werden in neuen herbiziden Wirkstoffkombinationen gemeinsam mit Fotosynthesehemmer-Herbiziden eingesetzt (DE-OS 33 32 272).

Weiterhin können durch eine dem Fachmann bekannte hydrolytische Spaltung gleichzeitig tert.-Alkylamine ("Ritter-Amine") und gegebenenfalls substituierte Pyridincarbonsäuren erhalten werden. Pyridincarbonsäuren werden bei spielsweise zur Synthese von Malariamitteln vom Typ des Isonikotinsäure-Hydrazids (Isoniacid) verwendet.

Beispiele 1 bis 20 (Allgemeine Arbeitsvorschrift)

1,0 Mol 2-Cyanopyridin wird mit 1,1 Mol tert.-Butanol unter den in der Tabelle 1 angegebenen Reaktionsbedingungen umgesetzt. Diese Reaktionsbedingungen umfassen wechselnde Mengen und Konzentrationen an $H_2SO_4$, wechselnde Mengen an verschiedenen (Halogen)Kohlenwasserstoffen sowie wechselnde Reaktionszeiten und -temperaturen. Das 2-Cyanopyridin und der (Halogen)Kohlenwasserstoff wurden jeweils vorgelegt, $H_2SO_4$ oder tert.-Butanol zusätzlich vorgelegt und die jeweils fehlende Komponente (tert.-Butanol bzw. $H_2SO_4$) innerhalb von 40 Minuten bei der angegebenen Reaktionstemperatur zugetropft. Die Tabelle 1 gibt an, welches diese zugetropfte Reaktionskomponente war. Der Reaktionsansatz wurde sodann bei der angegebenen Reaktionstemperatur solange nachgerührt, daß sich die in der Tabelle 1 angegebene Reaktionszeit ergab. Das Reaktionsgemisch wurde sodann auf Raumtemperatur gebracht und mit Natronlauge neutralisiert. Dabei erwärmte sich das Reaktionsgemisch. Die Dosiergeschwindigkeit der Natronlauge wurde so bemessen, daß sich eine Temperatur von 40° C einstellte. Die organische Phase wurde bei 40° C abgetrennt und über Natriumsulfat getrocknet. Danach wurde der verwendete (Halogen)Kohlenwasserstoff abgezogen und der Rückstand gaschromatographisch analysiert. Die Tabelle 1 enthält die hierbei ermittelten Prozent-Gehalte im Reaktionsprodukt an nichtumgesetztem 2-Cyanopyridin, Pyridin-2-carbonsäureamid (durch Abspaltung der tert.-Butylgruppe entstanden) und an dem gewünschten Pyridin-2-carbonsäure-N-tert.-butylamid.

Tabelle 1: Umsetzung von 1 Mol 2-Cyanopyridin (2-PyCN) mit 1,1 Mol tert.-Butanol (t-Bu-OH); (Hal)KW = (Halogen)Kohlenwasserstoff, 2-$PyCONH_2$ = Pyridin-2-carbonsäureamid, 2-PyCONH-Bu = Pyridin-2-carbonsäure-N-tert.-butylamid

| Nr. | (Hal)KW Art | Menge (ml) | $H_2SO_4$ Konz. (%) | Menge (Mol) | Dosierung | Reaktions- -zeit (h) | -temp. (°C) | %-Gehalt im Produkt an *) 2-PyCN | 2-$PyCONH_2$ | 2-PyCONH-Bu |
|---|---|---|---|---|---|---|---|---|---|---|
| 1 | $CH_2Cl_2$ | 50 | 100 | 2,0 | t-Bu-OH | 3 | 20 | | 4,0 | 94,0 |
| 2 | $CH_2Cl_2$ | 50 | 100 | 2,0 | $H_2SO_4$ | 1 | 20 | | 4,2 | 86,0 |
| 3 | $CH_2Cl_2$ | 50 | 90 | 2,0 | $H_2SO_4$ | 6 | 20 | 0,3 | 1,5 | 97,3 |
| 4 | $CH_2Cl_2$ | 50 | 96 | 2,0 | $H_2SO_4$ | 5 | 20 | 2,0 | | 96,1 |
| 5 | $CH_2Cl_2$ | 50 | 70 | 2,0 | $H_2SO_4$ | 24 | 20 | 62,3 | 0,5 | 34,0 |
| 6 | $CH_2Cl_2$ | 200 | 90 | 1,5 | $H_2SO_4$ | 168 | 20 | 27,8 | | 71,1 |
| 7 | $CH_2Cl_2$ | 200 | 90 | 1,5 | t-Bu-OH | 168 | 20 | 18,8 | 0,9 | 80,3 |
| 8 | $CH_2Cl_2$ | 50 | 90 | 2,0 | t-Bu-OH | 6 | 40 | | | 94,0 |
| 9 | $CH_2Cl_2$ | 50 | 84 | 2,0 | t-Bu-OH | 6 | 40 | | | 98,0 |
| 10 | $CH_2Cl_2$ | 200 | 90 | 1,9 | t-Bu-OH | 6 | 40 | | | 98,0 |
| 11 | ortho-Öl | 50 | 90 | 2,0 | t-Bu-OH | 3 | 50 | | | 98,0 |
| 12 | ortho-Öl | 50 | 90 | 1,5 | t-Bu-OH | 6 | 50 | | | 98,0 |
| 13 | Cl-benzol | 50 | 90 | 2,0 | t-Bu-OH | 3 | 50 | | | 98,0 |
| 14 | Cl-benzol | 50 | 90 | 1,0 | t-Bu-OH | 6 | 50 | 24 | - | 76 |
| 15 | Cl-benzol | 50 | 90 | 1,5 | t-Bu-OH | 6 | 50 | | | 98,0 |
| 16 | Toluol | 50 | 90 | 1,5 | t-Bu-OH | 6 | 50 | | | 98,0 |
| 17 | i-Octan | 50 | 90 | 1,5 | t-Bu-OH | 6 | 50 | | | 97,0 |
| 18 | Cyclohexan | 50 | 90 | 1,5 | t-Bu-OH | 6 | 50 | | | 97,0 |
| 19 | Cyclohexan | 50 | 90 | 1,1 | $H_2SO_4$ | 6 | 80 | 22 | 10 | 68 |
| 20 | Cyclohexan | 50 | 90 | 1,1 | t-Bu-OH | 6 | 80 | 9,1 | 2,4 | 88,1 |
| 21 | $CCl_4$ | 50 | 90 | 1.9 | t-Bu-OH | 2 | 50 | 2,1 | 2,5 | 93,8 |

*) Bei einigen Beispielen wurden geringe Mengen an Nebenkomponenten beobachtet, die nicht näher identifiziert werden

**Ansprüche**

1. Verfahren zur Herstellung von Pyridin-carbonsäure-N-tert.-alkylamiden der Formel

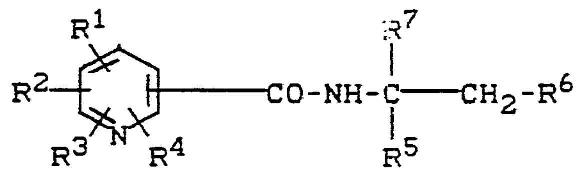

,

in der
$R^1$, $R^2$, $R^3$ und $R^4$ unabhängig voneinander Wasserstoff, Halogen, Alkyl, Alkoxy, Phenyl, Benzyl oder Nitro bedeuten und zwei der Reste $R^1$ bis $R^4$ gemeinsam einen anellierten Benzolring bedeuten können und $R^5$, $R^6$ und $R^7$ unabhängig voneinander geradkettige oder verzweigte Alkylgruppen mit 1-8 C-Atomen bedeuten, wobei weiterhin $R^5$ und $R^6$ gemeinsam mit den C-Atomen, die sie substituieren, einen aliphatischen Ring von 5-8 Ringgliedern bilden können, weiterhin $R^7$ ein gegebenenfalls substituierter Phenylrest sein kann und $R^6$ außerdem Wasserstoff sein kann,
durch Umsetzung eines Pyridincarbonsäurenitrils der Formel

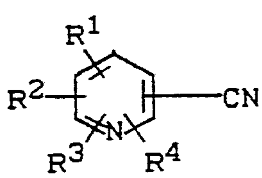

,

in der
$R^1$ bis $R^4$ die angegebene Bedeutung besitzen,
mit einem tertiäre Carbeniumionen liefernden Alkylierungsmittel der Formel

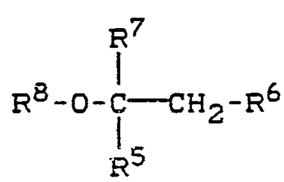

,

oder der Formel

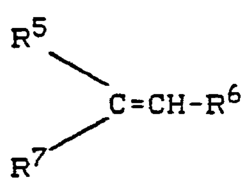

,

in denen
$R^5$ bis $R^7$ die angegebene Bedeutung besitzen und
$R^8$ Wasserstoff, Alkyl oder Acyl bedeutet,
in Gegenwart von Schwefelsäure, dadurch gekennzeichnet, daß die Reaktion bei 0-100° C in Gegenwart eines aliphatischen oder aromatischen (Halogen)Kohlenwasserstoffes durchgeführt wird.

2. Verfahren nach Anspruch 1, dadurch gekennzeichnet, daß man ein Nitril der Formel

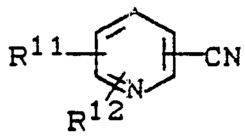

einsetzt, in der

$R^{11}$ und $R^{12}$ unabhängig voneinander Wasserstoff, Halogen, Alkyl, Alkoxy oder Nitro bedeuten.

3. Verfahren nach Ansprüchen 1 und 2, dadurch gekennzeichnet, daß man ein tertiäre Carbeniumionen lieferndes Alkylierungsmittel der Formeln

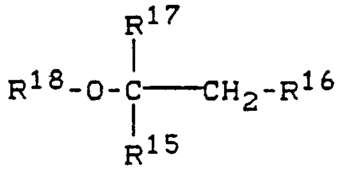

oder

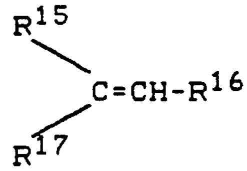

einsetzt, in denen

$R^{15}$ und $R^{17}$ unabhängig voneinander geradkettiges $C_1$-$C_4$-Alkyl bedeuten,

$R^{16}$ für Wasserstoff oder geradkettiges oder verzweigtes $C_1$-$C_4$-Alkyl steht und

$R^{18}$ Wasserstoff, $C_1$-$C_4$-Alkyl oder Acetyl bedeutet,

wobei weiterhin $R^{15}$ und $R^{18}$ gemeinsam mit den C-Atomen, die sie substituieren, einen aliphatischen Ring mit 5-6 Ringgliedern bilden können.

4. Verfahren nach Ansprüchen 1-3, dadurch gekennzeichnet, daß 10-2000 ml des (Halogen)-Kohlenwasserstoffs pro Mol des Pyridincarbonsäurenitrils eingesetzt werden.

5. Verfahren nach Ansprüchen 1-4, dadurch gekennzeichnet, daß 20-1000 ml des (Halogen)-Kohlenwasserstoffs pro Mol des Pyridincarbonsäurenitrils eingesetzt werden.

6. Verfahren nach Ansprüchen 1-5, dadurch gekennzeichnet, daß 30-300 ml des (Halogen)-Kohlenwasserstoffs pro Mol des Pyridincarbonsäurenitrils eingesetzt werden.

7. Verfahren nach Ansprüchen 1-6, dadurch gekennzeichnet, daß 40-200 ml des (Halogen)-Kohlenwasserstoffs pro Mol des Pyridincarbonsäurenitrils eingesetzt werden.

8. Verfahren nach Ansprüchen 1-7, dadurch gekennzeichnet, daß der eingesetzte (Halogen)-Kohlenwasserstoff mindestens teilweise vorgelegt oder mindestens teilweise gleichzeitig mit einem oder mehreren der Reaktionspartner zugesetzt wird.

9. Verfahren nach Ansprüchen 1-8, dadurch gekennzeichnet, daß das Pyridincarbonsäurenitril, das Alkylierungsmittel und der (Halogen)Kohlenwasserstoff vorgelegt werden und $H_2SO_4$ zudosiert wird.

10. Verfahren nach Ansprüchen 1-8, dadurch gekennzeichnet, daß das Pyridincarbonsäurenitril und der (Halogen)Kohlenwasserstoff vorgelegt werden, dann zunächst $H_2SO_4$ zugegeben wird und anschließend das Alkylierungsmittel zudosiert wird.